(19) Europäisches Patentamt
European Patent Office
Office européen des brevets

(11) **EP 4 372 751 A1**

(12) **EUROPEAN PATENT APPLICATION**
published in accordance with Art. 153(4) EPC

(43) Date of publication:
**22.05.2024 Bulletin 2024/21**

(51) International Patent Classification (IPC):
*G16B 40/20* (2019.01)

(21) Application number: **22857239.2**

(22) Date of filing: **08.03.2022**

(86) International application number:
**PCT/CN2022/079679**

(87) International publication number:
**WO 2023/019918 (23.02.2023 Gazette 2023/08)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **17.08.2021 CN 202110945074**

(71) Applicant: **Berry Oncology Co., Ltd.**
**Fuzhou, Fujian 350207 (CN)**

(72) Inventors:
• **ZHANG, Qingzheng**
**Fuzhou, Fujian 350207 (CN)**

• **ZHENG, Lu**
**Fuzhou, Fujian 350207 (CN)**
• **SUN, Fuming**
**Fuzhou, Fujian 350207 (CN)**
• **BAI, Jian**
**Fuzhou, Fujian 350207 (CN)**
• **WANG, Yin**
**Fuzhou, Fujian 350207 (CN)**
• **LI, Xiaoling**
**Fuzhou, Fujian 350207 (CN)**
• **WU, Lin**
**Fuzhou, Fujian 350207 (CN)**

(74) Representative: **Comoglio, Elena et al**
**Jacobacci & Partners S.p.A.**
**Corso Emilia 8**
**10152 Torino (IT)**

(54) **CANCER DETECTION MODEL AND CONSTRUCTION METHOD THEREFOR, AND REAGENT KIT**

(57) A cancer detection model and a construction method therefor, and a reagent kit, relating to the technical field of cancer detection. The method comprises: performing whole genome sequencing on plasma free DNA to mine nucleosome distribution features, terminal sequence features, and fragment size distribution features that can be applied to cancer detection; constructing classification models of the three indicators to obtain prediction scores of each indicator for a sample; then integrating these scores using a logistic regression model, and adding copy number variation feature information to obtain an ultimate classification and prediction model. The cancer detection model significantly improves the efficiency and accuracy of cancer detection, requires a small amount of data for analysis, has low detection cost, can detect different cancers, and is applicable for analyzing and predicting tumors in various stages. In addition, the reagent kit can complete the detection of the indicators required by the detection model, such that the reagent kit can be innovatively applied in ctDNA detection in the field of cancers.

Figure 1

**Description**

**Field of the Invention**

The invention relates to the technical field of cancer detection, specifically, to a cancer detection model and its construction method and kit.

**Background of the Invention**

Malignant tumors have become one of the major public health problems that seriously threaten the health of people around the world, and the prevention and control situation is grim. The latest global cancer burden data report in 2020 shows that there are approximately 19.29 million new cancer cases and 9.96 million deaths worldwide, and cancer is the first or second leading cause of human death in 112 countries around the world. If the growth level in 2020 is maintained, it is predicated that the number of new cases worldwide will reach 28.4 million in 2040, an increase of 47% from 19.29 million cases in 2020, and there will be a net increase of 4.1 million new cases in 2040. This forecast is likely to increase further due to population growth and ageing. With the rapid development of the economy, the prevalence of many known cancer risk factors has also increased, including smoking, unhealthy diets, obesity epidemics, and physical inactivity. Therefore, in order to reduce the burden of cancer, there is an urgent needs for intervention measures at both the prevention and treatment levels of cancer in the medical field. In fact, whether cancer is detected early and late, there is a huge difference in the choice of cancer treatment, quality of life, economic costs, and prognosis. Therefore, early detection and treatment are currently the most effective means of cancer treatment.

The existing clinical commonly used tumor screening and detection methods mainly include imaging examination, serological examination and pathological diagnosis, etc., and these screening and detection methods all have certain technical limitations, such as: (1)The commonly used serological markers have unsatisfactory diagnostic sensitivity and specificity, and are susceptible to inflammation, causing temporary abnormal information, leading to false positives or false negatives; (2) Imaging examination could only detect tumor lesions with a diameter of more than 1 cm, and they are basically in the middle and late stages when discovered; (3) Pathological diagnosis is the gold standard for cancer diagnosis, but it requires a needle biopsy and is generally used to confirm the diagnosis of patients with suspected cancer. However, since liquid biopsy is not invasive and can be repeated at different stages of disease treatment, the current focus of precision diagnosis and treatment of tumors has shifted to liquid biopsy. Among the analytes in liquid biopsy, cfDNA (circulating cell-free DNA, cfDNA) is the most widely used. cfDNA is a DNA fragment from cells that is free in the blood circulatory system, which mainly comes from fragmented DNA during apoptosis, DNA fragments from necrotic cells, and exosomes secreted by cells. The most important type of cfDNA is circulating tumor DNA (ctDNA), which is a DNA fragment from the tumor genome that enters the blood circulatory system, and can be a potential marker for early tumor screening because of the following advantages: (1) carries tumor information, provides information about tumor size, and can also provide a comprehensive description of the tumor genome to reflect the disease development status; (2) deep sequencing can uncover intra-tumor heterogeneity and genetic mutations that only appear in some cells. At present, the main research and development directions of ctDNA used in tumor screening and detection include ctDNA mutation detection, epigenetics (methylation, hydroxyl-methylation), and multi-omics detection, etc.

ctDNA mutation detection. Judging from the current research progress, the main problem encountered in ctDNA mutation detection is that the content of ctDNA itself in the blood is relatively low, and it will be cleaned up in real time, which is only about 0.1% to 1% of free plasma DNA, and Secondly, if ultra-deep sequencing is used, its detection cost is also very expensive, so ctDNA point mutations are mostly used in companion diagnosis and medication guidance for advanced cancers. Currently, a typical study in the field of ctDNA mutation detection is TEC-Seq, with an average sequencing depth of 30,000X, which is an ultra-sensitive detection method. The authors first used this method to detect plasma samples from normal individuals and found no tumor-related mutations. The authors then used this method to detect plasma samples from 194 patients with four types of tumors (breast, colon, lung, ovarian cancer). ctDNA content is higher in advanced tumors. The detection rate of late-stage tumors (stage III and IV) is > 75%, and the detection rate of early-stage tumors is 62%.

Other physical and chemical properties of ctDNA that are different from cfDNA. According to the article "Enhanced detection of circulating tumor DNA by fragment size analysis" published in Science Translational Medicine, the fragment length of ctDNA is smaller than that of cfDNA. Based on this, liquid biopsy is performed through fragment-specific enrichment, which has been verified to detect tumors earlier than CT in many cancer types. In this article, the authors did not include a cohort of early-stage tumor patients because the main application direction discussed in this article is not the screening and detection of early-stage patients, so there is no specific conclusion on the specific application of this method.

Based on the above situation, for early screening and early diagnosis of tumors, the sensitivity and specificity of each current research direction have their own advantages and disadvantages. Overall there is no particularly ideal result that can meet people's expectations.

In view of this, the present invention is hereby proposed.

**Summary of the Invention**

The object of the present invention is to provide a cancer detection model and its construction method and a kit.

The present invention is implemented in this way:

In a first aspect, embodiments of the present invention provide a method for constructing a cancer detection model, which includes: obtain the test data of each categorical metrics and copy number variation, and the categorical metrics included nucleosome footprint characteristics, end motif sequence characteristics, and fragment size distribution characteristics; use the test data of each categorical metrics as input data to construct a single-index classification model, and obtain the single-index prediction score of the sample; use the logistic regression model to integrate the single-index prediction scores of all categorical indicators to obtain the logistic regression scores for the samples; use the logistic regression score, copy number variation data, and single-index prediction scores of all categorical indicators as input data to construct cancer detection integrated model.

In a second aspect, embodiments of the present invention provide the use of reagents for detecting categorical metrics and copy number variations in preparing a kit for cancer detection, and the categorical metric is a classification index in the cancer detection model constructed by the construction method described in the previous embodiments.

In a third aspect, embodiments of the present invention provide a cancer detection kit for identifying cancer characteristics through whole-genome sequencing, which includes: a reagent for detecting categorical metrics and copy number variation, and the categorical metric is a classification index in the cancer detection model constructed by the construction method described in the previous embodiment.

In the fourth aspect, embodiments of the present invention provide a cancer detection model construction device, which includes: a data acquisition module, a prediction score acquisition module, a logistic regression score acquisition module and a cancer detection model building module;

Among them, the data acquisition module is used to obtain the categorical metrics and copy number variation test data of the sample to be tested, and the categorical metrics include nucleosome footprint characteristics, end motif sequence characteristics and fragment size distribution characteristics;

The prediction score acquisition module is used to input the test data of each categorical metric into a single-index classification model to obtain a single-index prediction score for the sample, and the single-index classification model is a single-index classification model constructed by the construction method described in the previous embodiment;

The logistic regression score acquisition module is used to input the single indicator prediction scores of all categorical indicators into the logistic regression module to obtain the logistic regression score of the sample;

The cancer detection model building are used to construct cancer detection models by using logistic regression scores, copy number variation data, and single-indicator prediction scores of all categorical indicators as input data.

In a fifth aspect, embodiments of the present invention provide a method for processing test data, which includes: obtaining test data for each categorical metrics and copy number variation of the sample, and where the categorical metrics includes nucleosome footprint characteristics, end motif sequence characteristics and fragment size distribution characteristics; correspondingly input the test data of each categorical metrics into a single-index classification model constructed by the construction method described in the previous embodiment, and obtain the single-index prediction score of the sample; enter the single-index prediction scores of all categorical indicators into the logistic regression model to obtain the logistic regression scores for the sample. Enter the logistic regression score, copy number variation data, and single-index prediction scores of all categorical metrics into the cancer detection model constructed by the construction method described in the previous embodiment.

In a sixth aspect, embodiments of the present invention provides a processing device for test data, which includes: a data acquisition module, a first execution module, a second execution module and a prediction module;

The data acquisition module is used to obtain the categorical metrics and the test data of copy number variation of the sample to be tested, and the categorical metrics include nucleosome footprint characteristics, end motif sequence characteristics and fragment size distribution characteristics;

The first execution module is used to input the test data of the categorical metrics into the single-index classification model constructed by the construction method described in the previous embodiment, and obtain the single-index prediction score to the sample;

The second execution module is used to input the single-index prediction scores of all categorical metrics s into the logistic regression model to obtain the logistic regression scores of the samples.

The prediction module is used to input the logistic regression scores, copy number variation data, and single-index prediction scores of all categorical metrics into a cancer detection model constructed by the construction method described in the previous embodiment, and obtain the prediction results of the sample.

In a seventh aspect, an embodiment of the present invention provides an electronic device, which includes: a processor and a memory, the memory is used to store one or more programs, and when the program is executed by the processor, the processor implements the method for constructing a cancer detection model as described in the previous embodiments, or the method for processing test data as described in the previous embodiments.

In an eighth aspect, embodiments of the present invention provides a computer-readable medium on which a computer program is stored, and when the computer program is executed by a processor, the method for constructing a cancer detection model as described in the previous embodiments is implemented, or a method for processing test data as described in the previous embodiment is implemented.

The present invention has the following beneficial effects:

Through whole-genome sequencing of plasma cell-free DNA, the present invention unearths multiple dimensions of the genomic features that can be applied to cancer detection (nucleosome footprint characteristics, end motif sequence characteristics, fragment size distribution). By constructing classification models for the indicators of these three features, the prediction score of each index for the sample is obtained, and then the logistic regression model is used to integrate these scores and add copy number variation feature information to obtain the final classification prediction model.

The cancer detection model constructed by the present invention can significantly improve the efficiency and accuracy of cancer detection, and the amount of data required for analysis is small. It only needs to meet the average sequencing depth of $0.25\times$ of the whole genome, and the cost and/or effect of detection exceeds existing methods., The method of the present invention is suitable for analyzing and predicting tumors at various stages and is especially suitable for the early detection of cancer.

The kit provided by the present invention can complete the detection of the indicators required by the detection model, so that it can be innovatively applied to the field of cancer in ctDNA detection.

**Brief Description of the Drawings**

In order to illustrate the technical solutions of the embodiments of the present invention more clearly, the drawings needed to be used in the embodiments will be briefly introduced below. And it should be understood that the following drawings only show some embodiments of the present invention and therefore should not be regarded as a limitation the scope. For those of ordinary skill in the art, other relevant drawings can also be obtained based on these drawings without exerting creative efforts.

Figure 1 is a flow chart of the construction of the cancer detection model in embodiment 1;
Figure 2 is a distribution diagram of nucleosomes corresponding to different groups in embodiment 1;
Figure 3 shows the proportion of different types of motif among different samples in embodiment 1;
Figure 4 shows the results of plasma low-depth WGS analysis of tumor patient and healthy people in embodiment 1;
Figure 5 is the detection data of the fragment size distribution characteristics between different samples in embodiment 1;
Figure 6 is a histogram of the statistical AUC results of the clinical indicators (AFP) related to liver cancer using the integrated model in embodiment 2 to compare the classification results of liver cancer;
Figure 7 is the calculated AUC value of the model constructed using an integrated model and a single classification index obtaining through data mining in embodiment 2;
Figure 8 shows the prediction scores of the integration scores for tumor patients at different BCLC stages in embodiment 3;
Figure 9 is a comparison between the integrated model in embodiment 4 of the present invention and the results of model construction using SVM after directly combining the four mined classification indicators.;
Figure 10 shows the prediction results of the integrated model for pancreatic cancer samples in embodiment 5.

**Detail Description**

In order to make the objectives, technical solutions and advantages of the embodiments of the present invention clearer, the technical solutions in the embodiments of the present invention will be clearly and completely described below. If no specific conditions are specified in the embodiment, the conditions should be carried out in accordance with conventional conditions or conditions recommended by the manufacturer. If the manufacturer of the reagents or instruments used is not indicated, they are all conventional products that can be purchased commercially.

Most of the existing studies only study a single index for early tumor detection, and rarely conduct joint research from multiple dimensions. Moreover, when multiple tumor detection indicators are known, the detection results that can be achieved by existing technology still have low sensitivity, low specificity or expensive detection cost, etc.

In terms of data analysis, the present invention analyzes the data from multiple dimensions, perform dimensionality reduction and feature extraction on the original feature data, defines reasonable model corresponding parameters, uses the grid search method to select the optimal parameter combination, and obtains an excellent classification model (Combine model) through iterative training, and define the integrated scoring rules, and finally uses the verification set and test set to verify the model effect.

Compared with the existing technology, it has the advantages of high sensitivity, good specificity, low detection cost, and is suitable for promotion.

Specifically, the method for constructing a cancer detection model provided by the embodiment of the present invention included the following steps:

Obtain test data of each categorical metrics and copy number variation, and the categorical metrics includes nucleosome footprint characteristics, end motif sequence characteristics and fragment size distribution characteristics; The test data of each categorical metrics is used as input data to construct a single-index classification model, and the single-index prediction score of the sample is obtained.

The logistic regression model is used to integrate the single-index prediction scores of all categorical metrics, and the logistic regression scores of the samples are obtained.

The logistic regression score, copy number variation data, and single-index prediction scores of all categorical indicators are used as input data to construct an integrated model for cancer detection.

## Definitions

Nucleosome footprint characteristics (NF): whole-genome sequencing of cfDNA isolated from plasma revealed that the distribution of nucleosomes in cfDNA is closely related to nuclear structure, gene structure, and gene expression observed in cells. In the promoter region of actively transcribed gene (House Keeping), the chromosome structure is relatively loose and the nucleosomes are sparsely distributed, while the phenomenon does not exist in silent gene (Silent), and the chromosome structure is relatively compact, and the nucleosome is more uniform and dense. Therefore, gene expression can be inferred based on differences in Nucleosome Footprint, because DNA that is not protected based on difference in nucleosome distribution, because during the process of DNA degradation in tissue cells and entering the blood, DNA that is not protected by nucleosomes is more likely to be degraded and less likely to be detected. Therefore, when sequencing cfDNA, fewer DNA molecules will be detected in the central region of the promoter of an active gene than in the surrounding region, which will lead to a reduction in the sequencing depth of the central region of the promoter of the gene, so nucleosomes can be quantified according to this principle. The same tumor cells should also have unique nucleosome footprint characteristics, which can be used as indicators for tumor detection.

According to the promoter region, the central region and the marginal region are defined, and the central region is 120-170 bp before the transcription start site (TSS) to 30-70 bp after the transcription start site (TSS);The edge region is the center region with edges extending outward on both sides 1800~2200bp.

The number of detected DNA fragments is counted for each region, which is used to calculate test data for the nucleosome footprint characteristics. Preferably, the test data of the nucleosome footprint characteristics is the nucleosome footprint difference value, and the nucleosome footprint difference value = the number of fragments in the marginal region / the total number of fragments (million) $\times$ the length of the marginal region (Kb) - the number of fragments in the central region / the total number of fragments $\times$ the length of the central region (Kb). This score indicates the distribution of nucleosomes and the transcriptional activity of genes, and is used as an input feature for model construction.

End Motif: the fragmentation of cfDNA is not a random event, especially for ctDNA released from tumor cells, which has a preferential fragmentation site, that is, specific fragmentation ends will be formed. Deep sequencing can find these tumor-specific broken ends, but is generally difficult to see with low-depth sequencing data. The present invention found that counting the proportion of base combination type at the ends of fragments can more effectively enrich this difference. The terminal sequence types that are significantly different from those of healthy people are found, and these motif with different proportions are used as input features for model construction.

Preferably, the test data of the motif sequence feature is the proportion of the differential terminal sequence, and the proportion of the differential motif sequence = (the type of base arrangement that is significantly different from the base arrangement at the end of the cfDNA fragment of the healthy sample / the sum of the terminal base arrangement types).

The "types of base arrangement with significant differences" can be confirmed in the following way: based on the distribution of terminal base arrangements in healthy samples to be tested, the proportion of each terminal base arrangement is counted, and when the proportion of a terminal base arrangement is significantly different between the healthy sample and the sample to be tested, the terminal base arrangement is marked as a terminal base arrangement with significant difference.

"Significant difference" in this article can refer to the case of $P<0.05$, or $P<0.01$, i.e., the difference is statistically significant.

Preferably, the terminal base arrangement refers to the arrangement of the last 3~6 bases at the end of the cfDNA fragment, which can be the arrangement of 3, 4, 5 or 6 bases.

More preferably, it is 4. The difference in distribution of the terminal 4 bases can more effectively reflect the difference between tumor samples and healthy samples, thereby achieving more accurate and effective detection of tumor samples. When the terminal base are arranged is the arrangement of 4 bases at the end (terminal 4mer bases), the total number of the terminal base arrangements is $4 \times 4 \times 4 \times 4 = 256$. The numbers of other bases can be deduced in the same way and will not be described again.

Fragment size distribution characteristics (Fragmentation) : The fragment length of cfDNA in cancer patients is shorter than that of cfDNA in healthy people, and the ratio of short fragments to long fragments of cfDNA in cancer patients is unstable compared with that of non-tumor individuals, and there are also differences in different regions of the genome.

Preferably, the test data of the fragment size distribution feature is the proportion of fragment differential distribution, and the proportion of fragment differential distribution = (Number of fragment size distribution difference regions / total number of divided regions), wherein the fragment size distribution difference region refers to the divided region where the proportion of short fragments and long fragments is significantly different compared with healthy samples, and the divided region refers to the region obtained by dividing the sample genome according to a specific length. The length of the long fragment > 150 bp, and the length of the short fragment is less than 150 bp.

Preferably, the specific length is 0.5-3M, and preferably 1M.

The "prediction score" in this article can be selected from the probability of a sample suffering from cancer and the cancer classification result output by the classification model.

The cancer detection model provided by the present invention has no special limitations on the type of cancer, and under the condition that the construction method remains unchanged, different cancers can adopt the samples of the corresponding cancer types to construct the model, and the parameters may correspond to slight changes, so as to predict and analyze the samples of different cancers more specifically.

The type of cancer can be selected from all known cancers, according to the location of the tumor, cancer can be divided into laryngeal cancer, brain cancer, esophageal cancer, stomach cancer, lung cancer, breast cancer, liver cancer and rectal cancer, etc. According to the tissue source of cancer cells, the cancer is divided into the following categories: adenocarcinoma, squamous cell carcinoma, large cell carcinoma, small cell carcinoma, carcinoid carcinoma, etc. According to the degree of differentiation of cancer cells, cancer it is divided into: well-differentiated cancer, moderately differentiated cancer, poorly differentiated cancer and undifferentiated cancer, etc.

Preferably, the construction method uses any classification algorithm among support vector machine (SVM), random forest, multilayer perceptron, elastic network, decision tree and deep neural network to construct a classification model for each classification index, SVM is preferred.

Preferably, the application also includes using the feature dimensionality reduction method to perform dimensionality reduction and feature extraction on the original data of each classification index, defining a reasonable corresponding parameters of the classification model, using the network search method to select the optimal parameter combination, and obtaining iterative training to get an excellent classification model.

Optionally, the feature dimensionality reduction method is selected from: any one of LASSO, PCA (principal component analysis), elastic network, random forest, and recursive elimination, preferably LASSO.

In the construction method provided in the embodiment of the present invention, the test data of the categorical metrics is obtained based on the sequencing data of the sample, and the sequencing can be high-depth sequencing, medium-depth sequencing or low-depth sequencing, preferably low-depth sequencing, preferably 0.25~5× sequencing, preferably 0.25×.

The logistic regression model plots the ROC curves of the single-indicator integrated model in the same coordinate to visually identify the advantages and disadvantages, and the ROC curve near the upper left corner represents the most accurate prediction. The performance of the model is quantified using AUC, which can also be compared by directly comparing the size of the AUC.

Preferably, the formula of the logistic regression model is shown in Table 1.

Preferably, the construction method includes using the data from the validation set and the test set to evaluate and optimize the performance of the classification model.

During optimization, the prediction score corresponding to a single classification index is multiplied by the weight corresponding to each input feature, and the feature items is multiplied by the weights and summed with the intercept items. During training, the weight value will be further adjusted according to the size of the error until the iteration will stop when the number of iterations reaches the specified number or the error is less than a certain threshold.

Preferably, the construction method also includes mapping the summed result to [0, 1] using the sigmoid function, that is, converting the result into a percentage.

Copy number variation (CNV): The healthy human genome is diploid, and the tumor tissue genome will produce abnormal copies during the replication process, so the tumor tissue genome will produce copy number amplification or deletion in certain chromosomes or certain regions, that is, copy number variation, and the CNV signal in tumor tissue can be detected in plasma, and the signal can still be stably detected down to an average of $1\times$ genome coverage depth.

Preferably, copy number variation data can be obtained in the following way: divide the sample genome into several regions according to a certain length (1~5K, preferably 2K), count the average sequencing depth of each small region, and perform GC correction; use the data of healthy samples to construct a negative pool to calculate the average GC-corrected sequencing depth of the obtained healthy samples in each small region as a baseline. Compare the tumor samples with the negative pool to find small areas where the copy number differences exceeds a specific threshold, and perform connect to form the final region with copy number variation. Calculate the CNV score based on the type of copy number variation (amplification and deletion) and the proto-oncogenes and tumor suppressor genes involved in the region, and the specific formula is shown in Table 1.

The present invention takes the test data of copy number variation (CNV score) as a qualitative index, and further integrates the three categorical metrics and the model results after using logistic regression integration through the above-mentioned set rules to from an integrated cancer detection model which can detect cancer more accurately, and has a very positive effect on improving the cure rate of mild cancer patients and the survival time of severe patients.

Preferably, the construction method includes integrating the single-index prediction scores corresponding to all categorical indicators to obtain single-index scores, and then using them as input data together with logistic regression scores and copy number variation data for the construction of cancer detection models. That is, the cancer detection model score includes three parts: a single index score, a logistic regression score, and a copy number variation score.

As long as the technical solutions for outputting sample prediction results are implemented through machine learning methods and based on the single index score, logistic regression score and copy number variation score, they all fall within the scope of protection of the present application.

Preferably, the scoring rules for the integration of cancer detection models are as follows: the copy number variation feature score is 1 point for the area where the copy number change is detected to exceed a certain threshold (0.03-0.15); The logistic regression score is to use the logistic regression algorithm to integrate the Nucleosome Footprint characteristics, terminal sequence characteristics and fragment size distribution characteristics, and the score obtained is greater than its threshold and counted as 1 point. The score of each individual indicator is the sum of the scores of each individual indicator, and the score of each individual indicator is 0.5 points for the prediction score of each indicator obtained by the classification model of each indicator greater than its respective threshold. The scoring rules set in this application can improve the sensitivity and specificity of the detection more effectively.

Table 1 Calculation formula

| | Parameters | Charm$^{TSG}$ | Charm$^{OG}$ | Charm$^{Ess}$ |
|---|---|---|---|---|
| CNV Score | Calculation of parameters | $Charm_i^{TSG} = \sum_{j\|Gj\in Tj} Wj / N$ | $Charm_i^{OG} = \sum_{j\|Gj\in Oj} Wj / Nj$ | $Charm_i^{Ess} = \sum_{j\|Gj\in Essj} Wj / 1$ |
| | CNV Score formula | $CNV\ Score = Charm_i^{TSG} - \frac{\sum_i Charm^{TSG}}{\sum_i Charm^{OG}} \cdot Charm_i^{OG} - \frac{\sum_i Charm^{TSG}}{\sum_i Charm^{Ess}} \cdot Charm_i^{Ess}$ | | |
| Logistic Score | Genomic features of cfDNA | NF | Motif | Fragment |
| | Weight factors | X1 | X2 | X3 |
| | Logistic formula | Logistic Score = exp(Z)/(1+exp(Z)), where Z=-B+ (X1*NF) + (X2*Motif) + (X3*fragment) | | |
| Single Score | Single Score formula | $Single\ Score = \sum_{i\|i\in\{NF,Motif,Shmc,Fragment\}} 0.25 \times (sign(score_i - cutoff_i) + 1)$ | | |
| Combine Score | Combine Score formula | Combine Score = 0.5 × (sign(Logistic Score - cutoff$_{Logistic\ Score}$) + 1) + sign( CNV Score - cutoff$_{CNV\ Score}$) + Single Score | | |

Among them, logistic Score is the score of logistic regression, B is the intercept term, x1 is the NF weight, x2 is the Motif weight, x3 is the fragment weight, and Z is the sum of the sum of the feature items multiplied by the weights and the intercept term; Single Score is the score of a single indicator, and cutoff is the corresponding threshold; the integration score is the final prediction score of the sample; TSG is a tumor suppressor gene; OG is a proto-oncogene; ESS is a conventional functional gene, and i and j are chromosomal arms and genes of the human genome.

It should be noted that the corresponding parameters in the calculation formulas of the classification model, the logistic regression model and the integrated classification model are not limited to the setting of the above parameters, and the above corresponding parameters are preferred solutions. In other embodiments, the parameters can also be adjusted based on the data of the validation set and the test set.

As long as the construction of a cancer detection model or the processing of sequencing data is implemented based on the above technical concepts, it falls within the protection scope of the present invention.

The embodiments of the present invention also provide the application of a reagent for detecting categorical metrics and copy number variation in the preparation of a kit for cancer detection, and the classification index is a classification index in the cancer detection model constructed by the construction method described in any of the aforementioned embodiments.

The present invention does not specifically limit the specific types of reagent, and can be used to obtain the test data of sample categorical metrics and copy number variation.

The embodiments of the present invention also provide a kit for cancer detection, which includes: a reagent for detecting categorical metrics and copy number variation, and the categorical metrics is a classification index in the cancer detection model constructed by the construction method described in the aforesaid arbitrary embodiment.

The embodiments of the present invention also provide a construction device of cancer detection model, which includes: a data acquisition module, a prediction score acquisition module, a logistic regression score acquisition module and a cancer detection model building module.

Wherein, the data acquisition module is used for obtaining the test data of categorical metrics and copy number variation of the sample to be tested, and the categorical metrics comprises nucleosome footprint characteristics, motif sequence characteristics and fragment size distribution characteristics;

The acquisition module is used to input the test data of each categorical metrics into the classification model to obtain a single index prediction score for the sample, and the classification module is a classification model constructed by the construction method described in the preceding arbitrary embodiment;

The logistic regression score acquisition module is used to input the single indicator prediction scores of all categorical indicators into the logistic regression module to obtain the logistic regression score of the sample.

The cancer detection model building blocks are used to construct cancer detection models by using logistic regression scores, copy number variation data, and single-indicator prediction scores of all categorical indicators as input data.

The embodiments of the present invention also provide a method for processing test data, which includes:

Obtain the test data of each categorical metrics and copy number variation of the sample, and the categorical metrics includes nucleosome footprint characteristics, motif sequence characteristics and fragment size distribution characteristics;

The test data of each categorical metrics is correspondingly input into a classification model constructed by the construction method described in any embodiment described above, and the prediction score of the sample is obtained;

The predicted scores of all categorical indicators are input into the logistic regression model to obtain the logistic regression scores of the samples;

The data of logistic regression scores, copy number variation, and single-index prediction scores of all categorical indicators were input into the cancer detection model constructed by the construction method described in any of the preceding embodiments.

The data processing method does not have the direct purpose of diagnosing or treating the disease, but mechanically processes it based on the test data.

The embodiments of the present invention also provide a processing device for test data, which includes: a data acquisition module, a first execution module, a second execution module and a prediction module.

Wherein the data acquisition module is used for obtaining the categorical metrics and the test data of copy number variation of the sample to be tested, and the classification index comprises nucleosome footprint characteristics, motif sequence characteristics and fragment size distribution characteristics;

The first execution module is used for inputting the test data of the categorical metrics into the classification model constructed by the construction method as described in any of the previous embodiment of to obtain a single index prediction score for the sample;

The second execution module is used to input the single-indicator prediction scores of all categorical indicators into the logistic regression model to obtain the logistic regression scores on the samples;

The prediction module is used for inputting logistic regression scores, copy number variation data, and single-index prediction scores of all categorical indicators into a cancer detection model constructed by the construction method described in any embodiment described above, and obtaining the prediction results of the sample.

The embodiments of the present invention also provide an electronic device includes: a processor and a memory for storing one or more programs, and when the program is executed by the processor, the processor implements a method for constructing a cancer detection model as described in any of the previous embodiments, or a method for processing test data as described in any of the previous embodiments.

The electronic device may include memory, processor, bus, and communication interface, which are directly or indirectly electrically connected to each other to enable data transmission or interaction. For example, these components can be electrically connected to each other via one or more bus or signal lines. The processor may process information and/or data relating to the identification of the object to perform one or more of the functions described in this application. For example, the processor can obtain an image to be identified and perform target recognition based on the data, thereby realizing the object recognition method provided in the present application.

Memory can be but is not limited to, Random Access Memory (RAM), Read Only Memory (ROM), Programmable Read-Only Memory (PROM), Erasable Programmable Read-Only Memory (EPROM), Electric Erasable Programmable Read-Only Memory (EEPROM), etc.

The processor can be an integrated circuit chip with signal processing capabilities. The processor can be a general-purpose processor, including a Central Processing Unit (CPU), a Network Processor (NP), etc; it can also be a Digital Signal Processing (DSP), a dedicated integrated circuit (Application Specific Integrated Circuit, ASIC), Field-Programmable Gate Array (FPGA) or other programmable logic devices, discrete gate or transistor logic devices, and discrete hardware components.

In practical applications, the electronic device can be a server, a cloud platform, a mobile phone, a tablet, a laptop, an ultra-mobile personal computer (UMPC), a handheld computer, a netbook, a personal digital assistant (PDA), a wearable electronic device, a virtual reality device and other devices. Therefore, the embodiment of the present application does not limit the type of electronic equipment.

The embodiments of the present invention also provides a computer-readable medium on which a computer program is stored, and when the computer program is executed by a processor, the method for constructing a cancer detection model as described in any of the preceding embodiments is implemented, or a method for processing test data as described in any of the foregoing embodiments is implemented.

Computer readable media include: USB flash drive, mobile hard disk, read-only memory, random access memory, magnetic disk or optical disk and other media that can store program code.

The features and performance of the present invention will be described in further detail below with reference to embodiments.

**Embodiments**

**Embodiment 1**

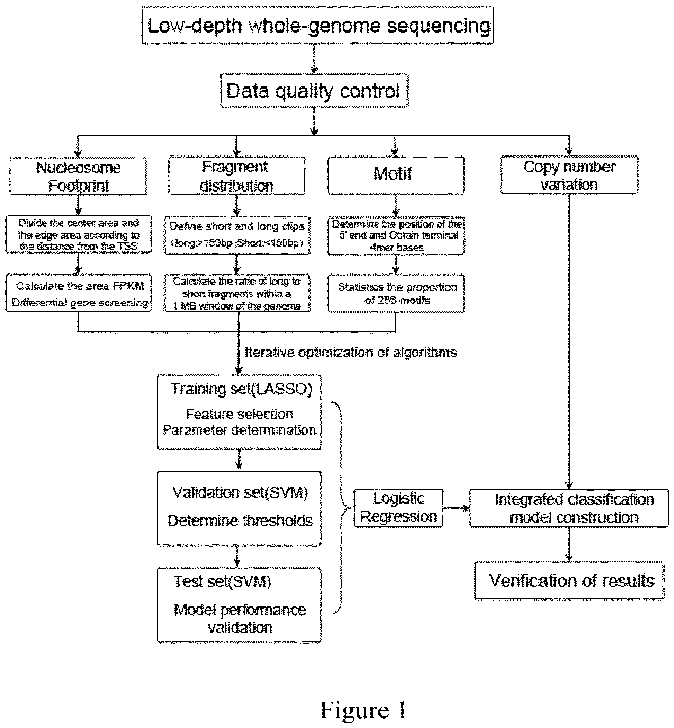

Referring to Figure 1, this embodiment provides a method for constructing a cancer detection model, which includes the following steps.

1. cfDNA extraction and low-depth WGS sequencing.

Plasma separation: Peripheral blood is collected in a non-invasive anticoagulant tube, and sample information and sample status are recorded and checked. The blood collection tube is placed in a centrifuge for centrifugation, the supernatant is aspirated and centrifuged again, and the supernatant is aspirated again to obtain a plasma sample.

Extraction of cell-free DNA (cfDNA) from blood: plasma cell-free DNA is extracted with a plasma cell-free DNA extraction kit. After the plasma is completely melted, centrifuge it at high speed, and transfer the centrifuged plasma into a tube. Add lysis buffer, magnetic beads and other reagents respectively. Shake, mix and incubate for a period of time, and then use cleaning solution to wash, and use the elution buffer to recover DNA.

DNA quality detection: DNA concentration is measured with a Qubit nucleic acid/protein quantitative fluorometer or microplate reader.

WGS library preparation and sequencing: Library is prepared using a whole-genome sequencing library preparation reagents, which mainly includes two steps: end repair (end repair buffer, end repair enzyme) and adapter ligation (adapter ligation buffer, adapter ligase, index adapter).

End repair buffer, end repair enzyme, ligation buffer, ligase and Index adapter are stored at -20 + 5 °C. Take them out to 2-8 °C to thaw and mix thoroughly before use. The magnetic beads need to be taken out from the 4 °C refrigerator in advance and equilibrated at room temperature for 30 minutes.

The end repair buffer and end repair enzyme premix are mixed with cfDNA and the reaction occurs at 37°C; the adapter ligation buffer and ligase premix are mixed with the end repair product of the previous step and the index adapter and the ligation reaction were performed at 20 °C. Purification is performed using purified magnetic beads after the adapter ligation.

The library is quantified by qPCR to see if the parameters such as the dissolution curve met the requirements, and the library concentration was calculated. Library quantification followed by on-machine sequencing. Use the qPCR method to quantitatively analyze the library, check whether the melting curve and other parameters meet the requirements, and calculate the library concentration. And the library is sequenced.

2. The integrated categorical metrics comes from the low-depth whole-gene sequencing data obtained by the above experimental method. Obtain sample data. The data used in this example are low-depth whole-genome next-generation sequencing (WGS) data of 481 patients diagnosed with liver cancer (HCC), 2247 patients with liver cirrhosis (LC), and 476 healthy controls (NC) recruited by the PreCar cohort project; Then conduct quality control and comparison of sequencing data, and conduct data mining of classification indicators.

3. Quality control and comparison of sequencing data.

Fastp software is used to filter the sequencing data, including subtracting the sequencing adapter sequence, removing the DNA fragments with sequencing read length less than 20, and removing DNA fragment with lower sequencing quality. Bowtie is used to compare the filtered data with the Hg19 reference genome to obtain the specific position information corresponding to each DNA fragment on the genome.

4. According to the results of the alignment, the data of nucleosome footprint characteristics (NF), end motif sequence characteristics (Motif) and fragment size distribution characteristics (Fragment) are obtained.

The test data of nucleosome footprint characteristics are nucleosome footprint difference values, and the nucleosome footprint difference value = the number of fragments in the marginal region/the total number of fragments (million)×the length of the marginal region (Kb) - the number of fragments in the central region/the total number of fragments×the length of the central region (Kb); Among them, the central region is 150 bp before the transcription start site (TSS) ~ 50 bp after the transcription start site (TSS); The edge area is the center area with edges extending outward by 2000bp on both sides;

The test data of the terminal sequence characteristics is the proportion of differential terminal sequences, and the proportion of differential terminal sequences = (the number of types of base arrangement that is significantly different from the arrangement of the last 4 bases at the end of the cfDNA fragment of a healthy sample/256);

The test data of the fragment size distribution characteristics is the proportion of fragment difference distribution, and the proportion of fragment difference distribution = (the number of fragment size distribution difference regions /the total number of divided areas), wherein the fragment size distribution difference region refers to the divided region where the proportion of short fragments and long fragments has significantly different compared with healthy samples, and the division region refers to the region obtained by dividing the sample genome according to a specific length.

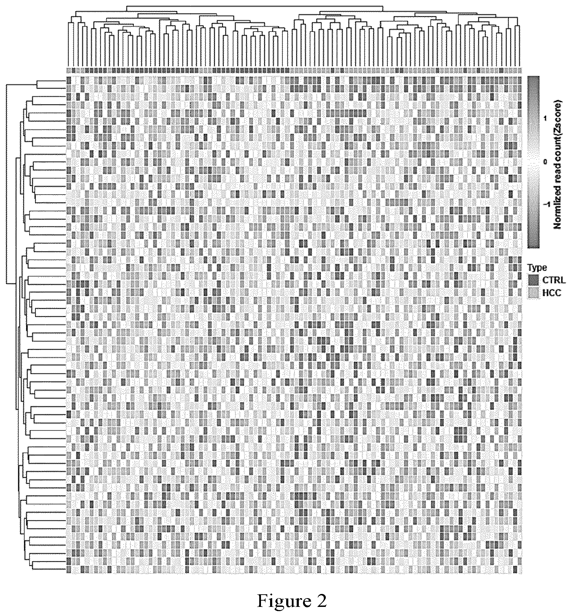

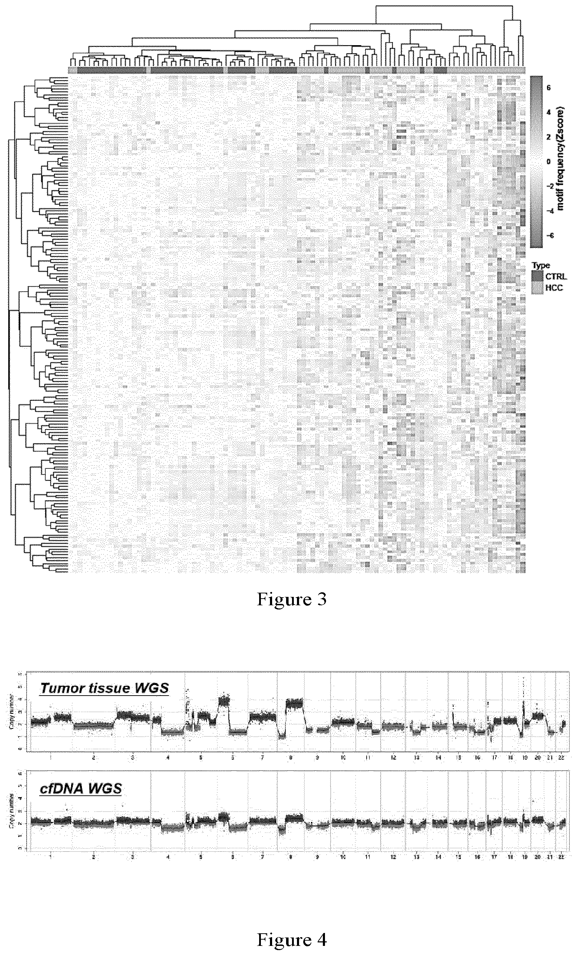

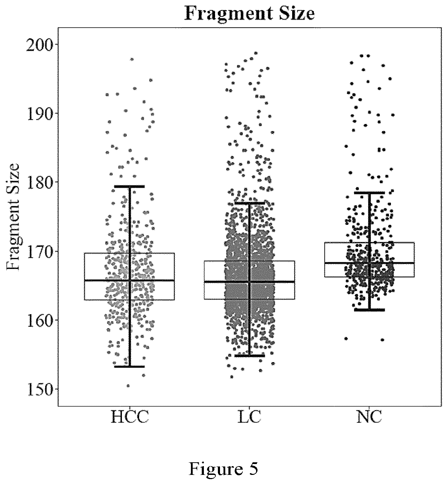

As shown in Figure 2-5, the distribution of nucleosomes varies between different groups (Figure 2); the proportions of different types of motif are also significantly different between different groups of samples (Figure 3). Low-depth WGS analysis of plasma from cancer patients shows significant changes in the copy number of certain segments of the genome compared with healthy people (cfDNA WGS) (Figure 4). The length of the ctDNA fragment in plasma will be shorter than the length of normal cfDNA fragment, defining the distribution region characteristics of the tumor (Figure 5). The above features are used as indicators for the construction of classification models.

5. The recruited samples were randomly divided into a training set, a validation set and a test set, and during the training process, the LASSO (least absolute shrinkage and selection operator) regression algorithm is used on the raw data of the above-mentioned three classification indicators to reduce the dimension of the data and extract relatively important features. For the extracted features, SVM is used to construct a classification model through cross-validation method to obtain the prediction score of a single indicator.

6. Use the single-index prediction scores of all classification indicators of the sample from the three classification models as input features, and use the logistic regression model to integrate the prediction scores of multiple metrics.

Use the validation set and test set data to evaluate the performance of the final model. The prediction score of the single-index classification model is the input item, and each input feature corresponds to a corresponding weight, and each feature item is multiplied by the sum of the weight and the intercept term to form the final output. During training, the weight value will be further adjusted according to the size of the error, until the number of iterations reaches the specified number or the error is less than a certain threshold. Finally, the summation result is mapped to [0, 1] using the sigmoid function, corresponding to the nucleosome footprint weight $x_1$ is 2.52. The motif sequence weight $x_2$ is 1.96, the fragment size distribution weight $x_3$ is 2.37, and the intercept term B is -4.55.

The calculation formula of the logistic regression model is as shown in Table 1.

7. Obtain the copy number variation data and refer to Table 1 for the calculation formula. The single-indicator prediction scores of all categorical indicators were integrated to obtain the single-indicator scores.

The single-index score, logistic regression score and copy number variation (CNV) data were used as input data, and set scoring rules, and build a cancer detection model (Combine model).

The calculation formula of the scoring rules is shown in Table 1, and the final integrated score contains three parts: copy number variation feature score, logistic regression score, and single index score. Among them, the copy number variation feature score is 1 point when the detected copy number change exceeds 0.03 in the region; the logistic regression score is to use the logistic regression algorithm to integrate the nucleosome footprint feature (NF), the motif sequence feature (motif) and the fragment size distribution feature (Fragment). If the obtained score is greater than its threshold, it will be counted as 1 point; the single indicator score is the sum of the scores of each individual indicator, and the score of each individual indicator is 0.5 points if the single indicator prediction score obtained by each indicator classification model is greater than its respective threshold.

## Embodiment 2

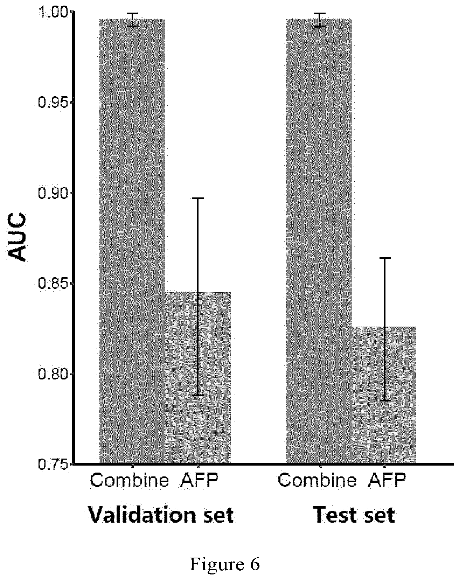

The construction method of embodiment 1 was used to construct the cancer detection model (Combine model), and the results of the cancer detection model (Combine model) and liver cancer-related clinical indicators (AFP) were used to calculated the AUC value and compare the classification results of the two classification results. (Figure 6).

As shown in the figure, the sensitivity and specificity of the traditional AFP detection are 53.77% and 90.81%, and the AUC is less than 0.85, while the sensitivity and specificity of the validation set of the present invention are 95.79% and 97.00%, the AUC is 0.996; the sensitivity and specificity of the the test set are 95.52% and 97.91%, and the AUC is 0.996 (the final result is based on effect), which greatly improves the detection accuracy.

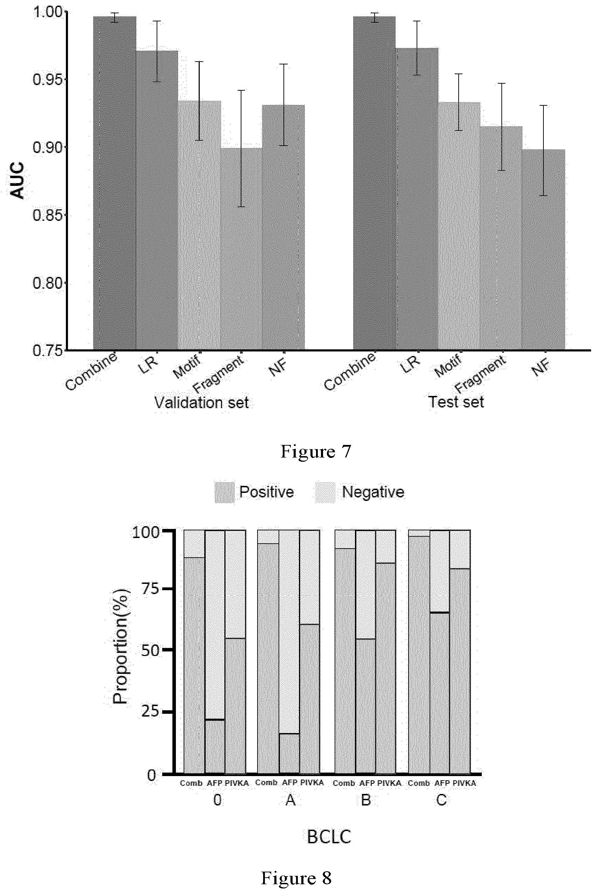

The AUC value is calculated using the results of the cancer detection model (Combine model) provided in embodiment 1 and the model constructed by a single categorical indicator obtained through data mining, and the results of the two types were compared, and the results are shown in Figure 7 and Table 2.

Table 2 Test results

| Hepatocellular carcinoma vs non-Hepatocellular carcinoma | | | | |
|---|---|---|---|---|
| | Validation set | | Test Set | |
| | Sensitivity | Specificity | Sensitivity | Specificity |
| Logistic regression | 92.63% | 97.00% | 93.13% | 96.56% |
| Motif | 89.47% | 84.00% | 77.10% | 92.07% |
| NF | 83.16% | 90.50% | 77.86% | 90.66% |
| Fragment size distribution | 83.16% | 84.00% | 83.97% | 83.46% |
| Combine | 95.79% | 97.00% | 95.42% | 97.91% |

According to the AUC of the model, it can be judged that the AUC of the Combine score is significantly greater than the AUC of other categorical indicators. It can be seen from the results that the sensitivity and specificity of the Combine model are also better than the performance of the model constructed with a single index, greatly improving the accuracy of detection.

## Embodiment 3

For tumor patients at different BCLC stages, there is basically no difference in the prediction score of the Combine model, therefore, it can be concluded that is suitable for each stage of the tumor (Figure 8). This result shows that the Combine model scoring method used in the present invention is significantly superior to the current clinical indicators.

## Embodiment 4

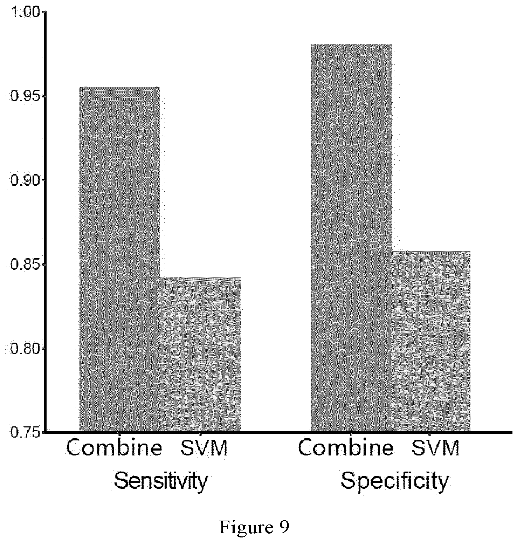

The present invention uses an integrated classification model (Combine model) and directly uses the four categorical metrics (nucleosome footprint characteristics, motif sequence characteristics, fragment size distribution characteristics, and copy number variation) to combine them together and then compare the results of model construction using SVM (Figure 9, The sensitivity is 0.841, the specificity is 0.858), and it can be seen from the results that the overall effect of the combine model is significantly better than the result of directly using the combination of four indicators and then using SVM to construct the model, further highlighting the obvious improvement effect on the classification results of the technical method of the present invention.

It should be noted that the sample information used in embodiments 2-5 is: the validation set includes 95 cases of liver cancer, 200 cases of non-liver cancer (100 cases of liver cirrhosis and 100 cases of healthy people); the test set includes 131 cases of liver cancer and 1916 cases of non-liver cancer (1800 cases of liver cirrhosis and 116 cases of healthy people).

**Embodiment 5**

The present embodiment provides a method for constructing a cancer detection model, which is roughly the same as embodiment 1. The difference is that pancreatic cancer samples are used to construct the model. The difference between obtained integrated model and the model in embodiment 1 lies in the parameters. In the model, the weights corresponding to each single indicator in the logistic regression formula change, specifically the nucleosome footprint weight of 1.458, the fragment size distribution weight of 1.1052, the motif sequence weight of 2.4305, and the value of the intercept term in the logistic regression formula changes to -2.2985.

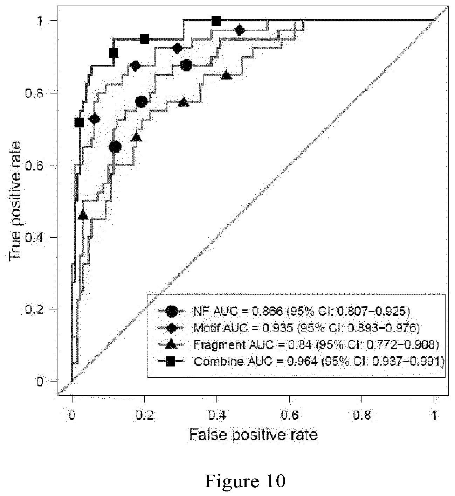

The obtained integrated model was used to classify pancreatic cancer samples, including 124 pancreatic cancer samples and 200 healthy individuals. The results are shown in Figure 10 and Table 3. As the number of samples increases, the subsequent improvement effect will be more obvious.

Table 3 Test results

| Pancreatic cancer vs Healthy people | | | |
|---|---|---|---|
| | AUC | Specificity | Sensitivity |
| Nucleosome footprint (NF) | 0.866 | 85.38% | 75.00% |
| Fragment size distribution (Fragment) | 0.84 | 82.31% | 70.00% |
| Motif sequence(Motif) | 0.935 | 82.50% | 90.77% |
| Combine | 0.964 | 94.62% | 87.50% |

The above descriptions are only preferred embodiments of the present invention and are not intended to limit the present invention. For those skilled in the art, the present invention may have various modifications and changes. Any modification, equivalent substitutions, improvement, etc., made within the spirit and principles of the present invention shall be included in the protection scope of the present invention.

**Claims**

1. A method for constructing a cancer detection model, **characterized in that** it includes:

   Obtain test data of each classification indicators and copy number variation, and the classification indicators includes nucleosome footprint characteristics, end motif sequence characteristics and fragment size distribution characteristics;
   The test data of each classification indicators is used as input data to construct a single-index classification model, and the single-index prediction score of the sample is obtained;
   The logistic regression model is used to integrate the single-index prediction scores of all classification indicators, and the logistic regression scores of the samples are obtained;
   The logistic regression score, copy number variation data, and single-index prediction scores of all classification indicators are used as input data to construct an integrated model for cancer detection.

2. The method for constructing the cancer detection model according to claim 1, wherein the test data of the nucleosome footprint characteristics is a nucleosome footprint difference value;

   The nucleosome footprint difference value = (number of fragments in marginal area / total number of fragments $\times$ length of marginal area) - (number of fragments in central area / total number of fragments $\times$ length of central

area); among them, the central area is 120-170 bp before the transcription start site (TSS) to 30-70 bp after the transcription start site (TSS), and the marginal area is the center area with edges extending outward by 1800~2200bp on both sides;

The test data of the end motif sequence characteristics are the proportion of differential end motif sequences;

The proportion of the differential end motif sequence = the number of cfDNA fragments that differed significantly from the types of terminal base arrangement of a healthy sample / the number of all cfDNA fragments;

The test data of the fragment size distribution characteristics is the proportion of the fragment difference distribution;

Fragment differential distribution proportion = the number of fragment size distribution difference regions / the total number of fragment division regions, wherein the fragment size distribution difference regions refer to the division regions with significant differences in the proportion of short fragments and long fragments compared with healthy samples, and the division regions refer to the regions obtained by dividing the sample genome by a specific length.

3. The method for constructing the cancer detection model according to claim 2, **characterized in that** the terminal base arrangement refers to the arrangement of the last 3~6 bases at the end of the cfDNA fragment;

Preferably, the specific length is 0.5-3M.

4. The method for constructing the cancer detection model according to claim 1, **characterized in that** the formula of the logistic regression model is as follows:

Logistic Score = exp (Z) / (1+exp (Z)), where Z= -B+(x1×NF)+(x2×Motif) + (x3×Fragment) ; Among them, Logistic Score is the logistic regression score, and Z is the sum of each feature term multiplied by their respective weights and the intercept term, B is the intercept term, x1 is the NF weight, x2 is the Motif weight, x3 is the Fragment weight, NF is the nucleosome footprint characteristics, Motif is the terminal sequence characteristics, and Fragment is the fragment size distribution characteristics;

Preferably, the construction method includes integrating the single-indicator scores corresponding to all categorical indicators to obtain a single-index score, and using the single-indicator score as input data for the construction of a cancer detection model;

The formula for calculating the single-indicator score is as follows:

$$Single\ Score = \sum_{i\,|\,i\in\{NF,Motif,Fragment\}} 0.25 \times (sign(score_i - cutoff_i) + 1)) \quad ;$$

Among them, Single Score is the score of a single indicator, and cutoff is the corresponding threshold;

Preferably, the calculation formula of the cancer detection model is as follows:

**Combine Score = 0.5 × (sign(LogisticScore - cutoff$_{LogisticScore}$) + 1) + sign(CNVScore - cutoff$_{CNVScore}$) + SingleScore;**

Among them, combine Score is the final prediction score of the sample;

Preferably, the formula for calculating the copy number variation (CNV score) is as follows:

$$Charm_i^{TSG} = \sum_{j\,|\,Gj\in Tj} Wj/Nj \qquad Charm_i^{OG} = \sum_{j\,|\,Gj\in Oj} Wj/Nj \qquad Charm_i^{Ess} = \sum_{j\,|\,Gj\in Essj} Wj/Nj$$

$$CNV\ Score = Charm_i^{TSG} - \frac{\sum_i Charm^{TSG}}{\sum_i Charm^{OG}} \cdot Charm_i^{OG} - \frac{\sum_i Charm^{TSG}}{\sum_i Charm^{Ess}} \cdot Charm_i^{Ess}$$

Among them, TSG is a tumor suppressor gene; OG is a proto-oncogene; ESS is a conventional functional gene, and i and j are chromosomal arms and genes of the human genome.

5. The application of the reagents for categorical metrics and copy number variation in preparing a kit for cancer detection, **characterized in that** the categorical metrics is a classification indicator in the cancer detection model constructed by the construction method according to any one of claims 1~4.

6. A cancer detection kit for identifying cancer characteristics through whole-genome sequencing, **characterized in that** it includes: a reagent for detecting categorical metrics and copy number variation, and the classification indicator is a classification index in the cancer detection model constructed by any one of the construction methods of claims 1~ 4.

7. A cancer detection model construction device, **characterized in that** it includes:

The data acquisition module used to obtain the categorical metrics and the test data of copy number variation of the sample to be tested, and the categorical metrics comprises nucleosome footprint characteristics, end motif sequence characteristics and fragment size distribution characteristics;
The prediction score acquisition module, used to input the test data of each categorical metrics into a single-index classification model to obtain a single-index prediction score for the sample, and the single-index classification model is constructed by the construction method as described in any one of claims 1~4;
The logistic regression score acquisition module used to input the single indicator prediction scores of all categorical indicators into the logistic regression module to obtain the logistic regression score of the sample;
The integrated model building block for cancer detection is used to construct cancer detection models using logistic regression scores, copy number variation data, and single-index prediction scores of all categorical indicators as input data.

8. A method for processing test data, **characterized in that** it includes:

The test data of each categorical metrics and copy number variation of the sample were obtained, and the categorical metrics included nucleosome footprint characteristics, end motif sequence characteristics and fragment size distribution characteristics;
The test data of each categorical metrics are correspondingly input into a single-index classification model constructed by the construction method as described in any one of claims 1~4, and the single-index prediction score of the sample is obtained;
The single-indicator prediction scores of all categorical metrics were input into the logistic regression model to obtain the logistic regression scores of the samples;
The logistic regression score, copy number variation data, and single-index prediction scores of all categorical indicators were input into the integrated model of cancer detection constructed by the construction method described in any one of claims 1~4.

9. A device for processing test data, **characterized in that** it includes:

The data acquisition module is used for obtaining the categorical metrics and test data of copy number variation of the sample to be tested, and the categorical metrics comprises nucleosome footprint characteristics, end motif sequence characteristics and fragment size distribution characteristics;
The first execution module is used for inputting the test data of the categorical metrics into the single-index classification model constructed by the construction method as any one of claims 1~4, and obtains the single-index prediction score of the sample;
The second execution module is used to input the single-index prediction scores of all categorical indicators into the logistic regression model to obtain logistic regression scores of the sample;
The prediction module is used for inputting logistic regression scores, copy number variation data, and single-index prediction scores of all categorical indicators into the cancer detection model constructed by the construction method as described in any one of claims 1~4, and obtaining the prediction results of the sample.

10. The electronic device, **characterized in that** it includes: a processor and a memory, the memory is used for storing one or more programs, and when the program is executed by the processor, the processor implements a method for constructing a cancer detection model as claimed in any one of claims 1~4, or a processing method for test data as claimed in claim 8.

11. A computer-readable medium with a computer program stored thereon, **characterized in that**, when the computer program is executed by a processor, a method for constructing a cancer detection model as claimed in any one of claims 1-4 is implemented, or a method for processing test data as claimed in claim 8 is implemented.

Figure 1

Figure 2

Figure 3

Figure 4

Figure 5

Figure 6

Figure 7

Figure 8

Figure 9

Figure 10

<div align="center">INTERNATIONAL SEARCH REPORT</div>

| | International application No. |
|---|---|
| | **PCT/CN2022/079679** |

**A.    CLASSIFICATION OF SUBJECT MATTER**

G16B 40/20(2019.01)i

According to International Patent Classification (IPC) or to both national classification and IPC

**B.    FIELDS SEARCHED**

Minimum documentation searched (classification system followed by classification symbols)

G16B

Documentation searched other than minimum documentation to the extent that such documents are included in the fields searched

Electronic data base consulted during the international search (name of data base and, where practicable, search terms used)

CNPAT, WPI, EPODOC, CNKI, IEEE, ISI: 核小体, 分布, 足迹, 末端, 拷贝数, 变异, 逻辑回归, 癌, nucleosome, distribution, terminal, sequence, fragment, size, copy, number, variation, logic, regression, cancer

**C.    DOCUMENTS CONSIDERED TO BE RELEVANT**

| Category* | Citation of document, with indication, where appropriate, of the relevant passages | Relevant to claim No. |
|---|---|---|
| PX | CN 113838533 A (FUJIAN BERRYONCOLOGY TECHNOLOGY CO., LTD.) 24 December 2021 (2021-12-24)<br>    claims 1-11 | 1-11 |
| Y | CN 113160889 A (TSINGHUA UNIVERSITY et al.) 23 July 2021 (2021-07-23)<br>    description, paragraphs [0108]-[0143] | 1-11 |
| Y | CN 112086129 A (SHENZHEN GENEPLUS MEDICAL SCIENCE EXAMINATION LABORATORY) 15 December 2020 (2020-12-15)<br>    description, paragraphs [0078]-[0080] | 1-11 |
| Y | CN 104866732 A (BEIJING DNALEAD TECHNOLOGY CO., LTD.) 26 August 2015 (2015-08-26)<br>    description, paragraphs [0022]-[0043], and formula 3 | 1-11 |
| A | CN 110272985 A (GUANGZHOU XIONGJI BIOLOGICAL INFORMATION TECHNOLOGY CO., LTD.) 24 September 2019 (2019-09-24)<br>    entire document | 1-11 |
| A | US 2017045519 A1 (SHANGHAI KEXIN BIOTECH CO., LTD.) 16 February 2017 (2017-02-16)<br>    entire document | 1-11 |

☐ Further documents are listed in the continuation of Box C.        ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **19 May 2022** | **27 May 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.

**PCT/CN2022/079679**

| Patent document cited in search report | | | Publication date (day/month/year) | Patent family member(s) | | | Publication date (day/month/year) |
|---|---|---|---|---|---|---|---|
| CN | 113838533 | A | 24 December 2021 | None | | | |
| CN | 113160889 | A | 23 July 2021 | None | | | |
| CN | 112086129 | A | 15 December 2020 | None | | | |
| CN | 104866732 | A | 26 August 2015 | None | | | |
| CN | 110272985 | A | 24 September 2019 | None | | | |
| US | 2017045519 | A1 | 16 February 2017 | AU | 2015251314 | A1 | 03 November 2016 |
| | | | | EP | 3134545 | A1 | 01 March 2017 |
| | | | | JP | 2017515107 | A | 08 June 2017 |
| | | | | CN | 106414769 | A | 15 February 2017 |
| | | | | WO | 2015161792 | A1 | 29 October 2015 |

Form PCT/ISA/210 (patent family annex) (January 2015)